Europäisches Patentamt

European Patent Office

Office européen des brevets

(19)

(11) Publication number: **0 053 046**
**B1**

(12) **EUROPEAN PATENT SPECIFICATION**

(45) Date of publication of patent specification: **21.11.84**

(51) Int. Cl.³: **A 61 K 35/16**

(21) Application number: **81305611.6**

(22) Date of filing: **26.11.81**

(54) Stabilisation of factor VIII activity in blood plasma.

(30) Priority: **26.11.80 US 210383**

(43) Date of publication of application:
**02.06.82 Bulletin 82/22**

(45) Publication of the grant of the patent:
**21.11.84 Bulletin 84/47**

(84) Designated Contracting States:
**AT BE CH DE FR GB IT LI NL SE**

(56) References cited:
**US-A-4 203 891**

**CHEMICAL ABSTRACTS, vol. 75, no. 23, 6th December 1971, page 137, abstract 138539n, Columbus, Ohio, US, R. HOHAGE et al.: "Methodical studies on platelet aggregation in the rat. I. Applicability of recalcified citrate-heparin plasma in animal experimental studies"**
**DICTIONNAIRE VIDAL, 1979, Paris, FR, "Calciparine", pages 316-318**

**The file contains technical information submitted after the application was filed and not included in this specification**

(73) Proprietor: **Rock, Gail Ann**
**270 Sandridge Road**
**Rockcliffe Park Ottawa Ontario K1L 5A2 (CA)**

(72) Inventor: **Rock, Gail Ann**
**270 Sandridge Road**
**Rockcliffe Park Ottawa Ontario K1L 5A2 (CA)**

(74) Representative: **Lawrence, Peter Robin Broughton et al**
**GILL JENNINGS & EVERY 53-64 Chancery Lane London WC2A 1HN (GB)**

## Description

This invention relates to the stabilisation of the Factor VIII activity that is normally present in fresh blood. It is well known to be desirable to stabilise the activity of Factor VIII, so as to permit its separation in high yields, but existing methods have always resulted in considerable loss of activity.

Whole blood is generally collected for transfusion purposes into an anticoagulant which functions by chelating calcium and thus lowers the physiological level of calcium ions in the blood. In order to obtain a preparation enriched in Factor VIII activity, the whole blood is then separated into packed red cells (PC) and plasma. Following this and within six hours of collection, the plasma containing the Factor VIII is frozen at very low temperature, preferably −80°C, in order to maintain Factor VIII levels at reasonably high concentrations. The speed is necessary since it is well known that, if kept at room temperature or indeed maintained under any condition other than the frozen state, the Factor VIII activity in whole blood or blood plasma produced from the whole blood rapidly decays.

Thus, for example Penick, G. C. and Brinkhous, K. M. reported in Relative Stability of Plasma Antihemophilic Factor (AHF) Under Different Conditions of Storage, (Am. J. Med. Sci. 232:434—442, 1956) that deterioration of AHF on storage tends to be slow but progressive, with about 30 to 60% of the initial AHF remaining after a 3-week storage period. These researchers studied several factors including the effects of initial plasma AHF level of the donor, care and collection of blood, various types of anticoagulants and the conditions of storage to determine the amount of AHF remaining in stored blood or plasma. It was found that the lower the temperature of storage the better the preservation of Factor VIII, but that even did not prevent loss of half of the activity after one month.

Peter Wolf reported in Studies of Temperature and pH Stability of Human Antihemophilic Factor (AHF) in Plasma and in a Concentrate (Brit. J. Haemat. 5:169—176, 1959) that loss of AHF in trisodium citrate plasma stored at +4°C varied appreciably for different plasma samples, the losses obtained varied from 35 to 55% and 50 to 80% after storage times of 72 and 120 hours.

A. E. Preston reported in The Factor VIII Activity in Fresh and Stored Plasma, (Brit. J. Haemat. 13:42—59, 1967) that plasma Factor VIII is labile. Fresh blood stored at 4°C lost its activity to a variable extent, reports on the exact loss differed considerably. By freezing the plasma at a temperature of a low of −20°C most of the Factor VIII activity could be preserved for long periods. This then provided a convenient method for a blook bank to keep stocks of plasma ready for emergency treatment of haemophilic patients. However, the process of freezing and later thawing plasma causes some loss of Factor VIII activity unrelated to the actual period of storage. Preston indicated that it was known that the Factor VIII activity of fresh plasma starts to decline between the 6th and 12th hour of storage and that freezing the plasma appeared to prevent further loss. The sooner plasma was frozen the better, since it took as much as six hours to be completely frozen to less than −20°C. On these grounds it was thought prudent to start the cooling of the plasma as soon as possible. Centrifugation at 4°C was recommended.

C. Vermeer et al in Contributions to the Optimal Use of Human Blood: VIII. Stability of Blood Coagulation Factor VIII during Collection and Storage of Whole Blood and Plasma (Vox. Sang. 31 (Suppl. 1): 55—67, 1976) described a two phased decay curve for Factor VIII. The bi-phasic curve indicated that in fresh plasma Factor VIII occurs in a stable and in a labile form. In cryoprecipitate from fresh plasma, these researchers detected the labile Factor VIII in a statistically significant amount. The amount of stable Factor VIII in fresh plasma could be found by extrapolation of the curve. These researchers indicated that 15 to 20 hours after the collection of blood, more than 50% of the initial Factor VIII can be obtained by cryoprecipitation, provided that the blood is stored between 10 and 20°C and a pH of 6.9. This two phase decay curve was further described by M. D. Pepper et al in Plasma Factor VIII, Variables Affecting Stability under Standard Blood Bank Conditions and Correlation with Recovery in Concentrates (Transfusion 18(6):756—760, 1978). This study was designed to evaluate the loss of Factor VIII activity, especially within the first 18 to 24 hours of collection under normal blood bank conditions. In this study under the heading RESULTS, the authors reported the loss of Factor VIII activity in plasma stored at 4°C in comparison with frozen plasma at −40°C. Factor VIII decayed with a double half life, and the greatest loss occurred within the first 4 hours after collection. The main loss of Factor VIII and statistical significance during storage at 4°C in the first 24 hours after collection were shown. The results of the study showed that Factor VIII decays at 4°C in a bi-phasic manner. A rapid initial decrease with a half life of approximately 5 hours is followed by a relatively stable phase, with a half life of approximately 96 hours.

In U.S. Patent No. 4,203,891 issued May 20th, 1980 to G. A. Rock there is described a method of greatly increasing the yield of Factor VIII obtained from whole blood, blood plasma or blood plasma fractions which involves maintaining the physiological concentrations of calcium and/or other ions in the whole blood or plasma components. This is achieved by collecting whole blood or blood plasma from a donor directly into an anticoagulant which does not alter the physiological concentration of calcium or other ions, preferably heparin, and recovering the Factor VIII by conventional recovery techniques.

Thus whereas normally whole blood is collected into bags containing a citrate or other calcium chelating anticoagulant in the process of US Patent 4,203,891 the blood is collected into bags containing, for instance, heparin. Thus in each of the examples blood was collected into a bag containing heparin and in a comparative example within example 4 the blood was collected into a bag containing both heparin and CPD (a calcium chelating anticoagulant). However it is shown that this combined anticoagulant is not as effective as the use of the heparin anticoagulant alone.

In an Abstract entitled "The Role of Calcium, Thrombin, and other Proteolytic Enzymes in Factor VIII Degradation" by G. Rock and D. Palmer published in Abstracts of Volunteer Papers, American Association of Blood Banks, 33rd Annual Meeting, November 7—12, 1980 it is stated that, inter alia, the addition of physiological levels of calcium to citrated heparinised plasma effectively prevented decay and maintained 100% of the activity (of Factor VIII) for 24 hours.

Neither the Abstract nor the oral presentation to which it related disclosed the technique used to obtain the citrated heparinised plasma or to add the calcium. However in view of the known techniques for obtaining plasma discussed in the preceding paragraph it would be expected that this plasma would be obtained by collecting whole blood into bags containing a combination of a citrate anticoagulant and heparin, followed by separation of plasma.

The invention provides a method for stabilising the Factor VIII activity of fresh blood, plasma obtained from fresh blood or plasma obtained by plasmapheresis, wherein the blood or plasma has been collected in the presence of a calcium chelating anticoagulant in the absence of heparin. In the invention stabilisation of Factor VIII activity is achieved by adding dissolved calcium to the blood or plasma in the presence of heparin at a time not more than 6 hours after collection of the blood or subsequent to plasmapheresis. Thus whereas the above quoted Abstract suggested that the blood should be collected into a combined anticoagulant, in the invention it is important to collect the blood into the calcium chelating anticoagulant alone. Also, whereas in the Abstract it is stated that calcium is added to plasma that has previously been treated with heparin in the invention the calcium and heparin may be added together as a solution of calcium and heparin.

Although calcium and heparin can be added to whole blood to stabilise Factor VIII activity in whole blood it is preferred, in the invention, to add the calcium and heparin to blood plasma. Accordingly a preferred method according to the invention for stabilising Factor VIII activity in plasma obtained from whole blood or by plasmapheresis is characterised in that the dissolved calcium is added, in the presence of heparin, to plasma obtained by plasmapheresis in the presence of a calcium chelating agent in the absence of heparin or to plasma obtained from whole blood collected into a calcium chelating agent in the absence of heparin.

As a result of the invention it is possible to stabilise the Factor VIII activity substantially entirely in a method that incorporates conventional techniques of collection of whole blood. Thus these conventional techniques require the collection of whole blood into bags containing citrate or other calcium chelating anticoagulant. Prior proposals, such as in US Patent 4,203,891, would have required a change in this conventional practice. The method of the present invention however merely requires that, after the conventional collection step, calcium and heparin should be added to the plasma. Thus by the invention it is possible not only to obtain high yields of Factor VIII but to obtain them in a manner that is very easily carried out in conventional practice. Further, these high yields of Factor VIII are obtainable without the immediate freezing of the whole blood or blood plasma that is associated with conventional techniques of collecting Factor VIII. Instead the activity, in the invention, can be maintained for 24 hours or more, giving ample time for transport of the collected blood or blood plasma to a laboratory at which the conventional separation techniques can be conducted.

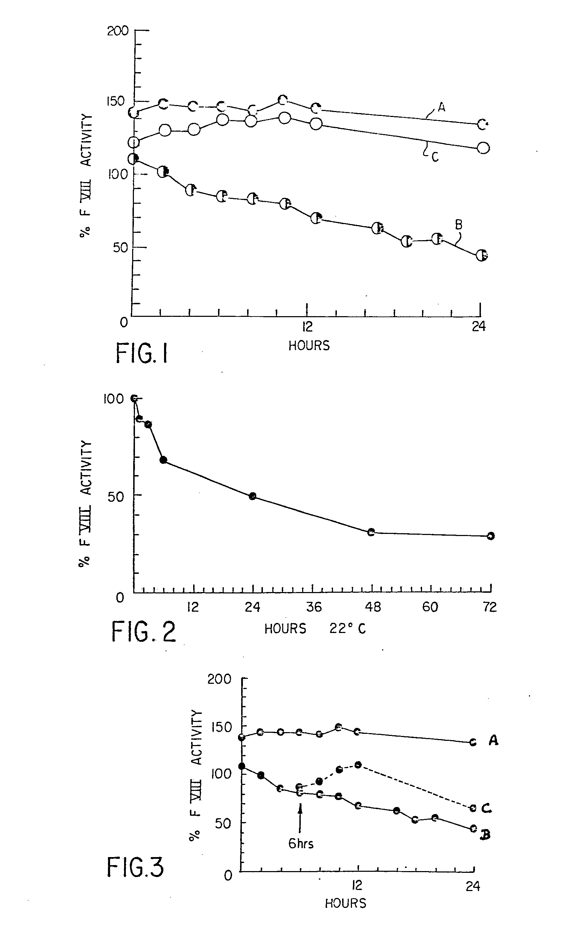

It is a surprising feature of the invention (for instance as demonstrated in Figure 3 below) that the addition of calcium in the presence of heparin can significantly restore Factor VIII activity that has been lost since blood collection, provided that the addition is effected within, at the most, 6 hours from collection.

It should be recognised that although the literature is full of reports of studies of ways of optimising the yield of Factor VIII for preparative procedures in fact none of these reports have led to processes that satisfactorily give high yields. Thus none of them have given any clear direction as to how to optimise the features necessary for improving the stabilisation of Factor VIII in such a way as to obtain consistently high qualitative and quantitative yields of Factor VIII. Thus the present invention is a significant step in the move towards obtaining high yields of Factor VIII in commercial practice. As an example, it should be noted that current methods of preparing Factor VIII generally result in only 15 to 20% of the initial levels of Factor VIII being recovered by the fractionator. This is largely because there is a loss of up to 50% of the Factor VIII activity by the time a cryoprecipitate is made and resolubilised. However the present invention permits retention of most, and sometimes all, of the Factor VIII activity before making the cryoprecipitate and will therefore permit recovery of much higher levels of Factor VIII.

As an indication of the importance of the recovery of Factor VIII in plasma fractionation, the estimated market price of Factor VIII in 1979 was approximately $.10 per unit. Now, it is estimated to be about $.15 per unit in the USA. However, the cost of Factor VIII preparations throughout the world varies greatly and an average price of $0.30 to $0.40 per unit is possible. In 1979, the estimates of the North American market requirements for Factor VIII were 720 million units which represents a value in excess of $50 million dollars. It is estimated that a single hemophiliac will require approximately 55,000 units per year in the USA for routine maintenance. Clearly any techniques which would aid in the commercialisation of methods for improving the yields of Factor VIII to meet market demands are of immeasurable significance.

In the invention fresh blood may be obtained in conventional manner in the presence of a calcium anticoagulant, for example by collection into a bag containing the anticoagulant, and plasma may be obtained from that blood in conventional manner. Alternatively plasma may be obtained by plasmapheresis in the presence of such an anticoagulant.

Anticoagulants which function by chelating calcium are well known in the art. These anticoagulants function by combining with, precipitating and effectively removing calcium ions normally present in the blood. They therefore reduce the concentration of calcium ion in the blood below normal physiological levels. Generally, the anticoagulants which fall within this definition include the citrate anticoagulants, for example, acid citrate dextrose (ACD), citrate phosphate dextrose (CPD), and trisodium citrate (TSC). Ethylenediaminetetraacetic acid (EDTA) may also be used. CPD is the most preferred anticoagulant of the available citrates.

The dissolved calcium and the dissolved heparin are then added to the citrated or chelated plasma or blood, the calcium can be introduced in the form of a solution of any acceptable soluble salt, for instance calcium chloride, calcium carbonate or calcium bicarbonate. The heparin is preferably introduced in the form of sodium heparin. Preferably the calcium and heparin are combined, and introduced as a single aqueous solution containing both calcium and heparin.

The amounts of calcium and heparin should be such as to be sufficient to stabilise the Factor VIII activity significantly. Generally this requires that the amount of calcium salt added to the blood plasma will be such that the calcium ion concentration is returned to near the normal physiological level, and preferably is such as to yield a free calcium ion concentration in the treated plasma of about 5 mg% (5 mg calcium per 100 ml plasma). The amount of heparin that is required for optimum stabilisation can easily be determined by routine experimentation but is generally in the range 2 to 20 units heparin per ml plasma, with best results generally being obtained with from 3 to 12, most preferably 4 to 8, units heparin per ml plasma. The amount is generally above 4 units heparin per ml.

Preferred compositions for addition to chelated plasma comprise a solution in water of heparin and soluble calcium compound. The derivatives of heparin and calcium mentioned above may be used. The composition will normally contain at least 80 units per 1 mg free calcium, but generally not more than 400 units. Preferred amounts of heparin are 120 to 360 units, and most preferably 80 to 160 units, heparin per 1 mg free calcium.

The amount of water introduced with the calcium and heparin should not be such as to dilute the plasma undesirably and generally the dilution is such that the total amount of water introduced into the plasma is less than 15% or 20%, and is preferably from 0.5 to 10%, by volume of the plasma. Thus a bag of 250 ml plasma may have 2 to 20 ml of the composition containing calcium and heparin added to it. The amount of water in the added composition may therefore be about 0.1 to 3 ml per 1 mg calcium and is usually below 5 ml per 1 mg calcium.

In a typical example a composition is prepared for addition to a bag of 250 ml chelated plasma. The composition has a volume of up to 20 ml and consists of 2250 units heparin and calcium chloride in an amount to give 5 mg% free calcium in the plasma.

In the drawings which are used to illustrate the present invention:

Figure 1 is a graph showing decay curves for Factor VIII activity;

Figure 2 is a typical two-phased decay curve showing Factor VIII activity in blood collected into CPD anticoagulant alone and

Figure 3 shows decay curves for Factor VIII activity and illustrates the effect of delayed addition of the heparin calcium mixture of the invention.

These graphs were obtained from a series of experiments, all of which were conducted at the same temperature. In Figure 1 curve A shows the percent Factor VIII activity at varying times from initial collection time for plasma prepared from blood collected directly into heparin anticoagulant alone, for instance as described in US Patent 4,203,891. Curve B is for blood collected into citrate phosphate dextrose (CPD) and heparin anticoagulant. The remarkable decrease in Factor VIII activity of curve B, compared to curve A, is immediately apparent.

Curve C is the curve obtained by the method of the invention and in particular by collecting blood into CPD, obtaining plasma from this and adding a solution of calcium chloride or other salt and heparin immediately after collection. The calcium and heparin are dissolved in a minimum quantity of water and are added to give from 4 to 8 units heparin per ml and 5 mg% calcium ion concentration. It is apparent that this results in substantial stabilisation of the activity of Factor VIII, and indeed results in some increase in activity in the early hours after addition. The activity in this particular experiment was not quite as high as when heparin was use alone but the use of heparin alone incurs some practical difficulties, as mentioned above.

The use of heparin alone as the anticoagulant provides a not unexpected result with respect to Factor VIII activity since it is not a chelating anticoagulant and as has been described earlier in U.S. Patent No. 4,203,891. issued May 20, 1980 to G. A. Rock, collection of blood directly into heparin does provide substantially improved levels of Factor VIII activity. Curve B is of course the typical decay curve one would expect when a combination of heparin and CPD anticoagulant are employed. The chelating effect of the CPD anticoagulant and hence the lower level of calcium is evident. The use of heparin does not counter the effect of the low calcium level. However, the result shown by curve C is quite unexpected and indicates clearly that the best results can be obtained when the addition of the heparin-calcium mixture is made immediately following collection of blood.

It should be noted that in Figure 3 the effects of the addition of the heparin-calcium mixture to blood plasma up to six hours after collection is illustrated. Curves A and B are the curves shown in Figure 1, while curve C is for plasma prepared from blood collected into CPD anticoagulant, treated with heparin and calcium six hours after the plasma was obtained (Heparin at 4—8 unit/ml plasma and sufficient calcium to give 5 mg% calcium ion concentration). It can be seen that there is initially some improvement in the Factor VIII activity even when the heparin-calcium addition is made six hours after collection. However, the storage properties when the addition is made at this time are not as good as when the addition is made immediately after collection.

The two-phase decay curve found in Figure 2 is the typical curve showing decay of Factor VIII activity in blood collected into CPD anticoagulant alone and as has been discussed earlier in the discussion of prior art. The amount of CPD anticoagulant in all these experiments may be conventional, e.g. 63 ml per 450 ml bag.

The main advantage associated with the method of the present invention is that Factor VIII activity can be maintained at levels normally present at the time of blood collection for periods of time up to and in excess of twenty-four hours. This will provide adequate time after blood collection for transportation of blood from collection centres to a central or main processing centre without the necessity of other preserving methods. Thus, the method permits the maintenance of Factor VIII activity and hence Factor VIII production from whole blood or blood plasma at high levels without any complicated or unusual preserving methods being required. In addition to this advantage, this method permits the separation of CPD blood and plasma prior to the heparin-calcium treatment of plasma, thus eliminating the need to remove heparin if blood had been initially collected into CPD and heparin anticoagulant.

Following the addition of the heparin-calcium mixture, any of the well known commercial or laboratory procedures for producing Factor VIII from plasma can be applied to the resulting product. These include various salting out processes such as ethanol extraction or polyethylene glycol treatment of the plasma or cryoprecipitation and treatment of the resulting cryoprecipitate with any of the usual agents such as ammonium sulphate, ethanol, polyethylene glycol or any other compound or technique generally used to obtain a preparation enriched in Factor VIII including application of a cold-precipitation step. The advantage is of course that such processes need not take place immediately in order to obtain high yields of Factor VIII.

**Claims**

1. A method for stabilising Factor VIII activity in fresh blood, blood plasma obtained from fresh blood or plasma obtained by plasmapheresis, the blood or plasma having been collected in the presence of a calcium chelating anticoagulant in the absence of heparin, characterised in that the Factor VIII activity is stabilised by adding dissolved calcium to the blood or plasma in the presence of heparin at a time not more than 6 hours after the collection of the blood or after the plasmapheresis.

2. A method according to claim 1 in which the amount of calcium that is added in the presence of heparin is an amount sufficient substantially to restore the level of calcium to a substantially normal physiological level.

3. A method according to claim 1 or claim 2 for stabilising Factor VIII activity in plasma obtained from whole blood or by plasmapheresis characterised in that the dissolved calcium is added, in the presence of heparin, to plasma obtained by plasmapheresis in the presence of a calcium chelating agent in the absence of heparin or to plasma obtained from whole blood collected into a calcium chelating agent in the absence of heparin.

4. A method according to claim 3 in which the amount of calcium added is sufficient to provide substantially 5 mg% free calcium ion concentration in the plasma.

5. A method according to claim 3 or claim 4 characterised in that the amount of heparin included in the plasma is from 2 to 20 units per ml plasma.

6. A method according to any of claims 3 to 5 in which the amount of heparin included in the plasma is from 4 to 8 units per ml plasma.

7. A method according to any of claims 3 to 6 characterised in that the total amount of water incorporated into the plasma is from 0.5 to 10% by volume of the plasma.

8. A method according to any preceding claim in which the calcium is added in the presence of heparin by adding a composition comprising calcium and heparin.

9. A method according to any preceding claim in which the amount of heparin that is added is from 80 to 360 units per mg of free calcium that is added.

10. A method according to any preceding claim in which the heparin is added in the form of sodium heparin.

11. A method according to any preceding claim in which the calcium chelating anticoagulant is citrate phosphate dextrose or acid citrate dextrose.

## Revendications

1. Un procédé pour la stabilisation de l'activité du facteur VIII dans du sang frais, de plasma sanguin obtenu à partir du sang frais ou du plasma obtenu par plasmaphérèse, le sang, ou le plasma, ayant été recueilli en présence d'un anticoagulant chélatant le calcium en l'absence d'héparine, caractérisé en ce que l'activité du facteur VIII est stabilisée par addition de calcium dissous au sang ou au plasma en présence d'héparine à un moment qui n'est pas situé à plus de 6 h après la récolte du sang ou après la plasmaphérèse.

2. Un procédé selon la revendication 1, dans lequel la quantité de calcium qu'on ajoute en présence de l'héparine est une quantité partiquement suffisante pour ramener le taux de calcium à un taux physiologique essentiellement normal.

3. Un procédé selon la revendication 1 ou la revendication 2, pour la stabilisation de l'activité du facteur VIII dans le plasma obtenu à partir du sang total ou par plasmaphérèse, caractérisé en ce que le calcium dissous, est ajouté, en présence d'héparine, à un plasma obtenu par plasmaphérèse en présence d'un agent chélatant le calcium en l'absence d'héparine, ou à un plasma obtenu à partir du sang total recueilli sur un agent chélatant le calcium en l'absence d'héparine.

4. Un procédé selon la revendication 3, dans lequel la quantité de calcium ajoutée est suffisante pour assurer essentiellement une concentration en ions calcium libres de 5 mg% dans le plasma.

5. Un procédé selon la revendication 3 ou la revendication 4, caractérisé en ce que la quantité d'héparine incorporée au plasma est de 2 à 20 unités par ml de plasma.

6. Un procédé selon l'une quelconque des revendications 3 à 5, dans lequel la quantité d'héparine incorporée au plasma est de 4 à 8 unités par ml de plasma.

7. Un procédé selon l'une quelconque des revendications 3 à 6, caractérisé en ce que la quantité totale d'eau incorporée au plasma est de 0,5 à 10% par rapport au volume u plasma.

8. Un procédé selon l'une quelconque des revendications précédentes, dans lequel le calcium est ajouté en présence d'héparine par addition d'une composition comprenant du calcium et de l'heparine.

9. Un procédé selon l'une quelconque des revendications précédentes, dans lequel la quantité d'héparine que l'on ajoute est de 80 à 360 unités par mg du calcium libre que l'on ajoute.

10. Un procédé selon l'une quelconque des revendications précédentes, dans lequel l'heparine est ajoutée sous forme d'héparine sodique.

11. Un procédé selon l'une quelconque des revendications précédentes, dans lequel l'anticoagulant chélatant le calcium est le citrate, phosphate, dextrose ou l'acide, citrate, dextrose.

## Patentansprüche

1. Verfahren zur Stabilisierung der Faktor VIII-Aktivität in Frischblut, aus Frischblut erhaltenem Blutplasma oder durch Plasmapherese erhaltenem Plasma, wobei das Blut oder das Plasma in Gegenwart eines Calcium-Chelierungs-Antikoagulierungsmittels in Abwesenheit von Heparin gesammelt worden ist, dadurch gekennzeichnet, daß die Faktor VIII-Aktivität durch Zugabe von gelöstem Calcium zu dem Blut oder Plasma in Gegenwert von Heparin zu einem Zeitpunkt, der nicht mehr als 6 Stunden nach der Sammlung des Blutes oder nach der Plasmapherese liegt, stabilisiert wird.

2. Verfahren nach Anspruch 1, worin die in Gegenwart von heparin zugesetzte Calciummenge eine solche Menge ist, die im wesentlichen zur Wiederherstellung des Calciumgehaltes auf ein in wesentlichen normales physiologisches Ausmaß ausreicht.

3. Verfahren nach Anspruch 1 oder Anspruch 2 zur Stabilisierung der Faktor VIII-Aktivität in aus Vollblut oder durch Plasmapherese erhaltenem Plasma, dadurch gekennzeichnet, daß das gelöste Calcium, in Gegenwart von Heparin, zu Plasma zugesetzt wird, das durch Plasmapherese in Gegenwart eines Calcium-Chelierungsmittels und in Abwesenheit von Heparin erhalten worden ist, oder zu Plasma zugesetzt wird, das aus Vollblut erhalten worden ist, das in ein Calcium-Chelierungsmittel in Abwesenheit von Heparin gesammelt worden ist.

4. Verfahren nach Anspruch 3, worin die zugesetzte Calciummenge ausreicht, um im wesentlichen eine Konzentration von 5 mg% freies Calciumion in dem Plasma zu ergeben.

5. Verfahren nach Anspruch 3 oder Anspruch 4, dadurch gekennzeichnet, daß die in das Plasma eingebrachte Heparin-menge von 2 bis 20 Einheiten je ml Plasma beträgt.

6. Verfahren nach einem der Ansprüche 3 bis 5, worin die in das Plasma eingebrachte Heparinmenge von 4 bis 8 Einheiten je ml Plasma beträgt.

7. Verfahren nach einem der Ansprüche 3 bis 6, dadurch gekennzeichnet, daß die in das Plasma eingebrachte Gesamtwassermenge von 0,5 bis 10 Vol.-% des Plasmas beträgt.

8. Verfahren nach einem der vorstehenden Ansprüche, worin das Calcium in Gegenwart von Heparin zugefügt wird durch Zugabe einer Calcium und Heparin enthaltenden Zusammensetzung.

9. Verfahren nach einem der vorstehenden Ansprüche, worin die zugesetzte Heparinmenge von 80 bis 360 Einheiten je mg zugesetztem freiem Calcium beträgt.

10. Verfahren nach einem der vorstehenden Ansprüche, worin das Heparin in Form Von Natrium-Heparin zugesetzt wird.

11. Verfahren nach einem der vorstehenden Ansprüche, worin das Calcium-Chelierungs-Antikoagulierungsmittel Citrat, Phosphatdextrose oder saure Citratdextrose ist.

0 053 046

FIG.1

FIG.2

FIG.3